# EUROPÄISCHE PATENTSCHRIFT

(11) **EP 1 236 740 B1**
(45) Veröffentlichungstag und Bekanntmachung des Hinweises auf die Patenterteilung: **18.07.2012**
(21) Anmeldenummer: 01104943.4
(22) Anmeldetag: 28.02.2001
(51) Int. Cl.: C07K 14/71, A61K 38/17

(54) **Vakzine gegen Krebserkrankungen, die mit dem HER-2/neu Onkogen assoziiert sind**
Vaccine against HER-2/neu oncogene-associated cancers
Vaccin contre cancers associés à l'oncogène HER-2/neu

(43) Veröffentlichungstag der Anmeldung: 04.09.2002
(73) Patentinhaber: Bio Life Science Forschungs- und Entwicklungsges.m.b.H., 1010 Wien (AT)
(72) Erfinder: Zielinski, Christoph, 1180 Wien (AT); Scheiner, Otto, 2380 Perchtoldsdorf (AT); Jensen-Jarolim, Erika, 1210 Wien (AT); Breiteneder, Heimo, 1070 Wien (AT); Prof. Dr. Ursula-Wiedermann, A-1130 Wien (AT)
(74) Vertreter: Kador & Partner

(56) Entgegenhaltungen:
- WO-A-01/08636
- WO-A-96/18409
- WO-A-99/57981
- JP-A- 05 117 165
- US-A- 5 801 005
- ZAKS: 'Cancer Res' Bd. 58, 1998, Seiten 4902 - 4908
- MURRAY SEMIN. ONCOL. Bd. 27, 2000, Seiten 71 - 75

### SEQUENZPROTOKOLL

<110> Bio Life Science Forschungs- und Entwicklungsges.
<120> Vakzine gegen Krebserkrankungen, die mit dem HER-2/neu Onkogen assoziiert sind
<130> K 36897
<140>
   <141>
<160> 2
<170> PatentIn Ver. 2.1
<210> 1
   <211> 17
   <212> PRT
   <213> Homo sapiens
<400> 1
   Cys Arg Val Leu Gln Gly Leu Pro Arg Glu Tyr Val Asn Ala Arg His
     1               5                  10                  15
   Cys
<210> 2
   <211> 14
   <212> PRT
   <213> Homo sapiens
<400> 2
   Tyr Met Pro Ile Trp Lys Phe Pro Asp Glu Glu Gly Ala Cys
     1               5                  10

## Beschreibung

Die vorliegende Erfindung betrifft eine Vakzine gegen Krebserkrankungen, die mit dem HER-2/neu Onkogen assoziiert sind.

In den letzten Jahren ist in den westlichen Industrienationen ein stetiges Anwachsen der Krebserkrankungen festzustellen, die damit eine der hauptsächlichen Todesursachen darstellen. Beispielsweise ist Brustkrebs die am weitesten verbreitete Krebsart bei Frauen, von dem etwa 10% aller Frauen in den westlichen Industrienationen betroffen sind.

Bisher bekannte Verfahren zur Therapie von Krebserkrankungen zielen vor allen Dingen auf eine frühe Erkennung der Erkrankung und auf operative Methoden bzw. eine möglichst selektive Abtötung der Tumorzellen durch Chemo- oder Radiotherapie ab. Diese Verfahren weisen die Nachteile auf, daß zum einen eine wirkungsvolle Prophylaxe gegen die Entstehung der Krebserkrankungen nicht möglich ist und daß zum anderen die Behandlung mit ganz erheblichen Nebenwirkungen für die Patienten verbunden ist.

Es ist weiterhin bekannt, daß es bei einer Vielzahl von Krebsarten wie beispielsweise Brust,- Eierstock,- Darm,- Lungen- und Prostatakrebs zu einer Überexpression des HER-2/neu-Proteins (auch als p185 oder c-erbB2 bezeichnet) als Proteinprodukt des HER-2/neu Onkogens kommt. Das HER-2/neu-Protein ist nicht nur mit dem malignen Phenotyp der Krebsart eng verbunden, sondern kann auch mit der Aggressivität der Krebsart verbunden sein. Das HER-2/neu-Protein ist ein Transmembranprotein mit einer relativen molekularen Masse von 185 kd und weist eine Länge von etwa 1255 Aminosäuren auf. Die Aminosäuresequenz des HER-2/neu-Proteins, sowie die Nukleinsäuresequenz einer dafür kodierenden DNA-Sequenz sind in US 5,869,445 wiedergegeben. Auf den Offenbarungsgehalt dieser Druckschrift wird hiermit Bezug genommen. Der extrazelluläre Teil des HER-2/neu-Proteins umfaßt die Peptidsequenz von Aminosäure 1 bis Aminosäure 675.

In US 5,869,445 wird ein Verfahren zur Stimulation einer Immunantwort auf das HER-2/neu-Protein offenbart, in dem ein Polypeptid der Sequenz von Aminosäure 676-1255 des HER-2/neu-Proteins verabreicht wird.

Es ist demgemäß Aufgabe der vorliegenden Erfindung, eine Vakzine gegen Krebserkrankungen, in denen es zu einer Überexpression des HER-2/neu-Proteins kommt, bereitzustellen, mit deren Hilfe es möglich ist, die Nachteile herkömmlicher Krebsbehandlungsmethoden zu vermeiden, eine wirksame Prophylaxe von solchen Krebserkrankungen zu ermöglichen und Alternativen zu dem aus US 5,869,445 bekannten Verfahren bereitzustellen.

Der Erfindung liegt die Erkenntnis zugrunde, daß eine solche Vakzine erhalten werden kann, wenn sie Teilstücke des extrazellulären Teils des HER-2/neu Proteins als wirksame Bestandteile enthält.

Gegenstand der vorliegenden Erfindung ist daher eine Vakzine gegen Krebserkrankungen, die mit dem HER-2/neu Onkogen assoziiert sind, gemäß Anspruch 1.

Mit der erfindungsgemäßen Vakzine ist eine aktive Immunisierung gegen Krebserkrankungen möglich, die mit dem HER-2/neu-Onkogen assoziiert sind. Somit kann eine Prophylaxe gegen diese Krebserkrankungen erhalten werden. Darüber hinaus kann die erfindungsgemäße Vakzine auch zur Therapie einer bereits bestehenden Krebserkrankung oder begleitend zu herkömmlichen Krebsbehandlungen eingesetzt werden. Durch die Anwendung der erfindungsgemäßen Vakzine können die oben beschriebenen erheblichen Nachteile herkömmlicher Krebsbehandlungsmethoden ganz oder teilweise vermieden werden.

Die erfindungsgemäße Vakzine enthält ein Peptid bestehend aus der Sequenzen von Aminosäure 544 bis 560 und ein Peptid bestehend aus der Sequenz von Aminosäure 610 bis 623 des extrazellulären Anteils des HER-2/neu-Proteins.

Die Sequenz des Peptids mit den Sequenzen von Aminosäure 544 bis 560 des extrazellulären Anteils des HER-2/neu-Proteins ist: CRVLQGLPREYVNARHC (Sequenz 1). Die Sequenz des Peptids mit den Sequenzen von Aminosäure 610 bis 623 des extrazellulären Anteils des HER-2/neu-Proteins ist: YMPIWKFPDEEGAC (Sequenz 2). Die Sequenzen sind im Sequenzprotokoll als Sequenznummer 1 bzw. Sequenznummer 2 wiedergegeben.
Fig. 1 zeigt einen Western-Blot der Immunpräzipitation eines Lysates von HER-2/neu überexprimierenden SKBR-3 Zellen mit Sera von Mäusen, die mit der erfindungsgemäßen Vakzine behandelt wurden, und unbehandelten Mäusen zum Nachweis der Immunantwort nach Verabreichung der Vakzine.

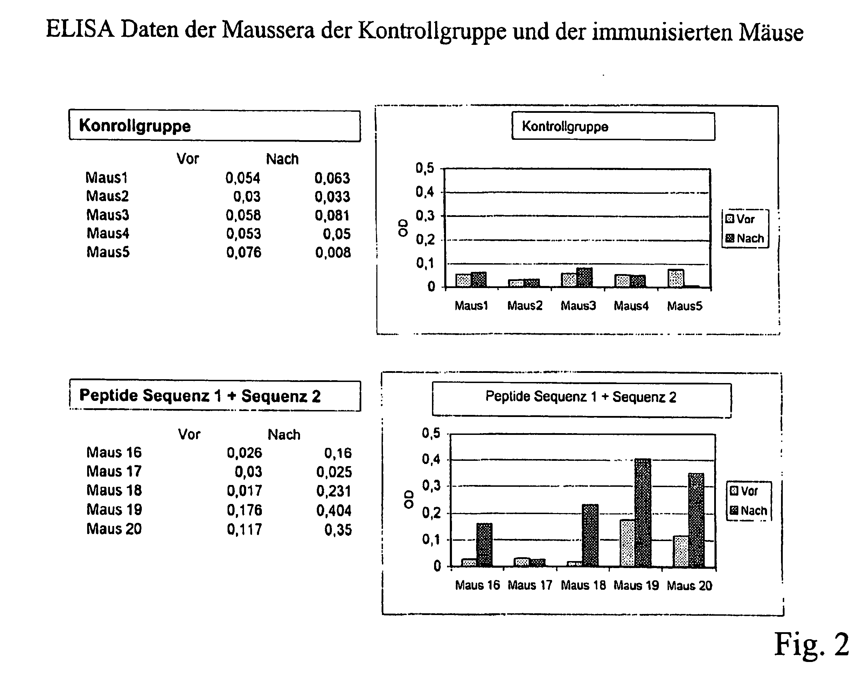

Fig. 2 zeigt ELISA-Daten der Sera von Mäusen, die mit der erfindungsgemäßen Vakzine behandelt wurden, und unbehandelten Mäusen zum Nachweis der Immunantwort nach Verabreichung der Vakzine.

Die erfindungsgemäßen Peptide können auch mit anderen Peptiden oder Polypeptiden oder mit weiteren chemischen Gruppen wie Glycosylgruppen, Lipiden, Phosphaten, Acetylgruppen oder ähnlichen verknüpft sein, soweit sie deren Wirkung nicht nachteilig beeinflussen.

Die erfindungsgemäße Vakzine enthält jedoch die Peptide, d.h. Teilstücke des extrazellulären Teils des HER-2/neu Proteins, mit den Sequenznummern 1 und 2.

In einer bevorzugten Ausführungsform werden die Peptide an einen immunogenen Träger konjugiert. Solche Träger können Makromoleküle aller Art sein. Die Konjugation mit einem Träger hat zur Folge, daß die Immunogenität der Vakzine erhöht wird.

Bevorzugt werden die Peptide an Keyhole Limpet Hemocyanin (KLH) oder Tetanustoxoid (TT) konjugiert.

Die Konjugation der Peptide an das Trägermaterial kann auf beliebige Weise erfolgen, beispielsweise auf gentechnologischem oder chemischen Weg, das heißt die Verknüpfung von Träger und funktioneller Gruppe erfolgt durch eine chemische Reaktion. Auf gentechnologischen Wege kann die Koppelung des Proteinträgermoleküls mit dem Peptid so hergestellt werden, daß eine für die Gesamtsequenz des Konjugats kodierende DNA- oder RNA-Sequenz in ein Expressionsystem eingebaut wird, von dem dann das Gesamtkonjugat exprimiert wird. Diese Form der Konjugation kann selbstverständlich nur für den Fall angewendet werden, daß auch das Gesamtkonjugat ein Proteinmolekül ist.

Bevorzugterweise werden die Peptide auf chemischem Weg mit dem Träger konjugiert. Das heißt, die Verknüpfung von Peptid und dem Träger zum Konjugat erfolgt auf chemischem Wege.

Die Peptide können als Mono,- Di,- Tri,- oder Oligomer mit dem Träger konjugiert werden. Solche Konjugationen sind beispielsweise in der Druckschrift von Th. H. Turpen, S. J. Reinl, Y. Charoenvit, S.L. Hoffman, V. Fallarme in Bio/Technology, 1995, Band 13, Seiten 53 bis 57 am Beispiel der Konjugation von Epitopen mit makromolekularen Trägern beschrieben. Die beschriebenen Vorgehensweisen lassen sich analog auf die Herstellung der Konjugate für die erfindungsgemäße Vakzine übertragen.

Wird die Konjugation eines di- oder oligomeren Peptidkonjugats auf dem Weg des oben beschriebenen gentechnologischen Verfahrens durchgeführt, so werden die für die Peptide kodierenden DNA- oder RNA-Abschnitte ein- oder mehrmals hintereinandergereiht in die für den Träger kodierende DNA- oder RNA-Sequenz integriert. Dadurch wird die Expression di- oder oligomerer Peptidkonjugate erreicht.

Die Mono- oder Oligomere der Peptide können sowohl in einfacher als auch in multipler Form an den Träger konjugiert werden, d.h. an einen Träger werden ein oder mehrere Peptidmoleküle angehängt.

Die erfindungsgemäße Vakzine kann auf verschiedene Arten appliziert werden. Die Verabreichung der die Peptide selbst enthaltenden Vakzine kann beispielsweise subkutan oder auch durch orale Einnahme der Vakzine in Kapsel oder Tablettenform erfolgen.

Die erfindungsgemäße Vakzine kann in vielfältiger Weise auf gentechnologischem oder chemischem Weg hergestellt werden. Solche Verfahren sind beispielsweise in US 5,869,445 beschrieben.

Ein Beispiel für ein gentechnologisches Herstellungsverfahren ist die Manipulation von Mikroorganismen wie E. coli. Diese werden so manipuliert, daß sie die Peptide als solche oder die Gesamtkonjugate bestehend aus Peptid und daran gekoppelten Träger exprimieren.

Bevorzugterweise werden die Peptide, auf chemischem Wege synthetisch dargestellt. In einer bevorzugten Ausführungsform geschieht dies mit Hilfe der Festphasensynthesemethode. Weiter bevorzugt wird das synthetisch dargestellte Peptid, auf chemischem Weg mit einem Träger wie KLH oder TT verknüpft.

Die erfindungsgemäße Vakzine kann zur prophylaktischen oder akuten Behandlung von Menschen und Tieren, die mit dem HER-2/neu Onkogen assoziierte Krebsarten entwickeln können, eingesetzt werden.

Im folgenden wird eine Ausführungsform der erfindungsgemäßen Vakzine, ein Verfahren zu deren Herstellung, sowie Methoden zum Nachweis von deren Wirksamkeit an einem Ausführungsbeispiel beschrieben.

### Beispiel

Die Peptide mit der Sequenznummer 1 und 2 wurden hergestellt und mit Keyhole Limpet Hemocyanin (KLH) konjugiert.

Die Peptide wurden mit der Festphasensynthese nach der FMOC-Schutzgruppen Strategie synthetisiert. Die Konjugation erfolgte über die Thiofunktion des C-terminalen Cysteins an das maleimidmodifizierte KLH.

### Immunisierung

Zum Nachweis der Wirksamkeit der erfindungsgemäßen Vakzine wurden zwei Gruppen zu jeweils fünf Mäusen folgendermaßen behandelt:

Der ersten Gruppe (Versuchsgruppe) wurden die beiden jeweils an KLH konjugierten Peptide mit den Sequenznummern 1 und 2, sowie Gerbu-Adjuvant verabreicht. Jede Injektion enthielt je 100 µg von jedem Konjugat in einem Volumen von 100µl gemischt mit 100µl Gerbu-Adjuvant, d.h. insgesamt 200 µl Antigenlösung pro Injektion. Die Kontrollmäuse wurden mit 200 µl einer Mischung von 100µl Wasser mit 100µl Gerbu-Adjuvant behandelt. Die Vakzine wurde s.c. in die Kniefalte verabreicht.

Gerbu-Adjuvant ist eine Adjuvansformulierung, die auf N-Acetylglucosaminyl-N-Acetylmuramyl-L-Alanyl-D-Isoglutamin basiert, mit Dimethyldioctadecylammoniumchlorid und einem Zink-L-Prolinkomplex als Synergisten. Dieses Adjuvans kann von der Firma Gerbu-Biotechnik GmbH, Gaiberg, Deutschland bezogen werden.

### Immunisierungsschema

Nach dem Priming erfolgten 3 Booster im Abstand je 3 Wochen. Sieben Tage nach dem letzten Booster wurden die Tiere getötet. Blut, Milz, Leber, Lunge und Nieren wurden entnommen.

Als Methoden zum Nachweis der Induktion einer Peptid- und HER-2/neu spezifischen Immunantwort in Mäusen wurden die folgenden drei verwendet:

### 1. Nachweis einer T-Zell Aktivierung in einem Proliferationsassay

Dabei wurden zunächst die aus den immunisierten Mäusen gewonnenen Milzzellen mit den zur Immunisierung verwendeten Peptiden der Sequenznummern 1 und 2 stimuliert. Als Maß für die Proliferation gilt der Einbau an ³H-Thymidin, der quantifiziert werden kann. Es werden die Counts pro Minute (cpm) ermittelt. Als Stimulationsindex (SI) bezeichnet man den Quotient aus cpm (mit dem jeweiligen Peptid stimulierter Zellen) / cpm (unstimulierter Zellen). In 96-Well Platten wurden 2 x 10⁵ Zellen pro Vertiefung fünf Tage lang mit den beiden Polypeptiden, mit der Sequenznummer 1 oder 2, in einer Konzentration von 20 µg/mL inkubiert. Nach dieser Zeit wurde ³H-Thymidin zugegeben und nach 18 Stunden Inkubation die eingebaute Aktivität gemessen.

Die oben beschriebene Prozedur erbrachte folgendes Ergebnis:

Der Stimulationsindex in der immunisierten Gruppe (Versuchsgruppe) variierte von 1.0 bis 1.6 für das Peptid der Sequenz 1 und von 1.5 bis 2.0 für Peptid der Sequenz 2.

Der Stimulationsindex in der Kontrollgruppe hatte in allen Fällen den Wert 1.0, d.h. es wurde keine Proliferation der Zellen festgestellt.

### 2. Nachweis HER-2/neu spezifischer Antikörper durch Immunpräzipitation

Da es kein kommerziell erhältliches HER-2/neu Protein gibt, wurde ein Zellysat von SKBR-3-Mammakarzinomzellen, die HER-2/neu überexprimieren, verwendet. Die Maussera wurden mit dem Zellysat inkubiert. Bei Vorhandensein spezifischer Antikörper im Serum wird das HER-2/neu aus dem Zellysat präzipitiert. Der gebildete Immunkomplex, kann der mit Hilfe von Protein A+G-Agarose (bindet Antikörper) isoliert werden. Diese Komplexe wurden dann durch eine 6%ige Polyacrylamid Gelelektrophorese (PAGE) aufgetrennt und auf eine Nitrozellulose-Membran transferiert. Das präzipitierte HER-2/neu wurde mit Hilfe eines kommerziell erhältlichen anti-HER-2/neu Antikörpers (Zymed) detektiert Dabei wird die entsprechende Bande auf der Membran durch eine Färbung visualisiert. Die hier beschriebene Methode dient als ein qualitativer Nachweis.

Die Analyse der Seren ergab folgendes Ergebnis:

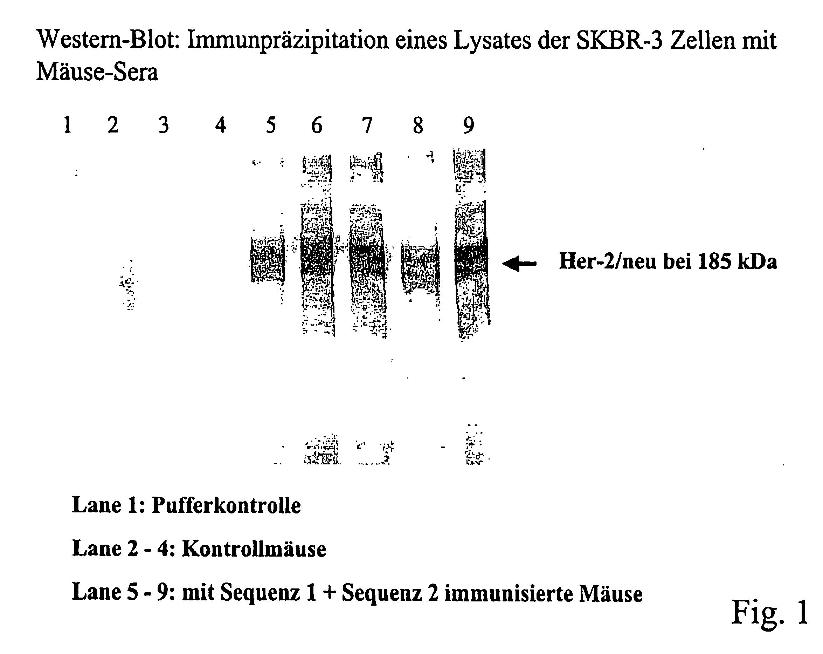

In Fig. 1 ist die Immunpräzipitation des Lysates der SKBR-3 Zellen mit Mäuse-Sera der Versuchs- und der Kontrollgruppe gezeigt. In allen Seren der Versuchsgruppe wurden die spezifischen anti HER-2/neu Antikörper nachgewiesen. Alle Seren der Kontrollgruppe zeigten eindeutig ein negatives Ergebnis.

### 3. Nachweis HER-2/neu spezifischer Antikörper mittels ELISA

Dabei wurden zunächst die Vertiefungen einer 96-Well ELISA-Platte mit Herceptin (anti-HER-2/neu-Antikörper von Genentech Inc.) beschichtet. Unpezifische Bindungsstellen wurden mit 2% Milch blockiert. Die Wells wurden dann mit den Membranfraktionen aus SKBR-3 Zellen, die das HER-2/neu-Protein überexprimieren, inkubiert. Um den unspezifischen Hintergrund zu quantifizieren, wurden Kontrollwells mit Membranfraktionen von HTB132-Zellen, die kein HER-2/neu-Protein exprimieren, inkubiert. In beiden Fällen wurden nachfolgend unspezifische Bindungsstellen mit 2% Milch abgesättigt ( blockiert).

Im nächsten Schritt wurden die Sera der immunisierten Mäuse (Maus 16 bis Maus 20) und die der Kontrollgruppe (Maus 1 bis Maus 5), die vor und nach der Immunisierung gewonnen worden waren, in einer Verdünnung von 1:100 in die Wells pipettiert. Für jedes Serum wurden 2 Wells mit SKBR-3-Membranen und 2 Wells mit HTB 132-Membranen verwendet. Gibt es im Serum spezifische Antikörper gegen das HER-2/neu-Protein, so werden sie an das Antigen binden. Diese Antikörper können dann mit Horseradish-Peroxidase (HRP)-gekoppelten sekundären Antikörpern detektiert werden. HRP geht mit dem Substrat eine Farbreaktion ein. Das Ergebnis dieser Farbreaktion kann mit dem Photometer gemessen werden. Dabei wird die Absorption bei 450 nm bestimmt und gegen den Hintergrund von 650 nm korrigiert (OD Werte). Als Ergebnis wird für jedes Serum die Differenz aus dem OD-Wert der beiden mit SKBR-3-Lysat inkubierten Wells minus dem OD-Wert der beiden mit HTB132-Lysat inkubierten Wells gewertet.

Diese Methode ermöglicht einen Vergleich des Antikörper-Titers in Seren.

Es wurden Seren aller Mäuse vor und nach der Vakzinierung ausgewertet. In 4 von 5 Mäusen der Versuchsgruppe ließ sich ein deutlicher Anstieg der OD-Werte nach der Immunisierung feststellen. Die erhaltenen OD-Werte der Kontrollgrupe zeigten keine Veränderung des Antikörper-Titers.

### Histopathologischer Befund

Lungen, Leber und Nieren wurden histopathologisch hinsichtlich Autoimmunreaktionen untersucht. In allen Fällen wurden Organe o.B. bewertet.

## Patentansprüche

1. Vakzine gegen Krebserkrankungen, bei denen es zu einer Überexpression des HER-2/neu Proteins kommt, **dadurch gekennzeichnet, dass** sie ein Peptid bestehend aus der Aminosäuresequenz (Sequenz Nr. 1) CRVLQGLPREYVNARHC, wie sie im extrazellulären Teil des HER-2/neu Proteins auftritt, und ein Peptid bestehend aus der Aminosäuresequenz (Sequenz Nr. 2) YMPIWKFPDEEGAC, wie sie im extrazellulären Teil des HER-2/neu Proteins auftritt, enthält.

2. Vakzine nach Anspruch 1, **dadurch gekennzeichnet, dass** die Peptide an einen immunogenen Träger konjugiert sind.

3. Vakzine nach Anspruch 2, **dadurch gekennzeichnet, dass** als Träger Keyhole Limpet Hemocyanin (KLH) oder Tetanustoxoid (TT) verwendet wird.

4. Vakzine nach einem der Ansprüche 2 oder 3, **dadurch gekennzeichnet, dass** die Konjugation chemisch erfolgt ist.

5. Vakzine nach einem der Ansprüche 2 bis 4, **dadurch gekennzeichnet, dass** die Peptide als Monomer, Dimer, Trimer oder Oligomer an die Träger konjugiert sind.

6. Vakzine nach einem der Ansprüche 2 bis 5, **dadurch gekennzeichnet, dass** die Peptide einfach oder mehrfach an den Träger konjugiert sind.

## Claims

1. A vaccine for cancerous diseases involving an overexpression of the HER-2/neu protein, **characterized in that** it contains a peptide consisting of the amino acid sequence (sequence no. 1) CRVLQGLPREYVNARHC as occurs in the extracellular portion of the HER-2/neu protein, and a peptide consisting of the amino acid sequence (sequence no. 2) YMPIWKFPDEEGAC as occurs in the extracellular portion of the HER-2/neu protein.

2. The vaccine according to claim 1, **characterized in that** the peptides are conjugated to an immunogenic carrier.

3. The vaccine according to claim 2, **characterized in that** as the carrier there is employed Keyhole Limpet Hemocyanin (KLH) or tetanus toxoid (TT).

4. The vaccine according to either claim 2 or 3, **characterized in that** the conjugation has been effected chemically.

5. The vaccine according to any of claims 2 to 4, **characterized in that** the peptides are conjugated to the carriers as a monomer, dimer, trimer or oligomer.

6. The vaccine according to any of claims 2 to 5, **characterized in that** the peptides are conjugated to the carrier singly or multiply.

## Revendications

1. Vaccin pour lutter contre des affections cancéreuses, dans lesquelles il se produit une surexpression de la protéine HER-2/neu, **caractérisé en ce qu'**il contient un peptide constitué de la séquence d'acides aminés (séquence n° 1) CRVLQGLPREYVNARHC, telle qu'elle apparaît dans la partie extracellulaire de la protéine HER-2/neu, et un peptide constitué de la séquence d'acides aminés (séquence n° 2) YMPIWKFPDEEGAC, telle qu'elle apparaît dans la partie extracellulaire de la protéine HER-2/neu.

2. Vaccin selon la revendication 1, **caractérisé en ce que** les peptides sont conjugués à un véhicule immunogène.

3. Vaccin selon la revendication 2, **caractérisé en ce que** l'on utilise, comme véhicule, l'hémocyanine de patelle (KLH) ou la toxoïde tétanique (TT).

4. Vaccin selon l'une quelconque des revendications 2 ou 3, **caractérisé en ce que** la conjugaison est effectuée par voie chimique.

5. Vaccin selon l'une quelconque des revendications 2 à 4, **caractérisé en ce que** les peptides sont conjugués au véhicule sous la forme de monomères, de dimères, de trimères ou d'oligomères.

6. Vaccin selon l'une quelconque des revendications 2 à 5, **caractérisé en ce que** les peptides sont conjugués une ou plusieurs fois au véhicule.
